(19) Europäisches Patentamt
European Patent Office
Office européen des brevets

(11) EP 4 613 111 A1

(12) EUROPEAN PATENT APPLICATION
published in accordance with Art. 153(4) EPC

(43) Date of publication:
**10.09.2025 Bulletin 2025/37**

(21) Application number: **23885768.4**

(22) Date of filing: **31.10.2023**

(51) International Patent Classification (IPC):
***A23L 33/10*** *(2016.01)* ***A61K 36/062*** *(2006.01)*
***A61K 36/07*** *(2006.01)* ***A61P 1/14*** *(2006.01)*
***A61P 43/00*** *(2006.01)*

(52) Cooperative Patent Classification (CPC):
**A23L 33/10; A61K 36/062; A61K 36/07; A61P 1/14; A61P 43/00**

(86) International application number:
**PCT/JP2023/039296**

(87) International publication number:
**WO 2024/096014 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.10.2022 JP 2022174336**

(71) Applicant: **House Foods Group Inc.**
**Higashiosaka-shi, Osaka 577-8520 (JP)**

(72) Inventor: **IWASAKI Yusaku**
**Kyoto-shi, Kyoto 606-8522 (JP)**

(74) Representative: **Schön, Christoph**
**Dr. Schön, Neymeyr & Partner mbB**
**Bavariaring 26**
**80336 München (DE)**

(54) **COMPOSITION FOR ACTIVATING TRANSIENT RECEPTOR POTENTIAL ANKYRIN 1, AND COMPOSITION FOR ENHANCING FOOD CONSUMPTION AND/OR AMELIORATING DECREASED APPETITE**

(57) The purpose of the present invention is to provide a novel TRPA1 agonist. The present invention relates to a composition for activating TRPA1, comprising a mushroom extract or lanthionine. The composition can enhance appetite and food consumption and ameliorate decreased appetite.



Processed by Luminess, 75001 PARIS (FR)

## Description

Technical Field

The present invention relates to a composition for activating transient receptor potential ankyrin 1 (TRPA1), and also relates to a composition for increasing food intake and/or ameliorating decreased appetite.

Background Art

TRPA1 is a type of nonselective cation channel belonging to the transient receptor potential (TRP) ion channel superfamily. TRPA1 is primarily expressed in somatosensory neurons (spinal sensory neurons and trigeminal neurons) and functions as a nociceptor involved in pain and pungency. TRPA1 is also abundantly expressed not only in somatosensory neurons but in afferent vagal nerves involved in feeding regulation.
Various compounds or plant extracts are known as agonists that activate TRPA1 (Patent Literatures 1-9). In particular, some pungent components in spices or pungent vegetables act as TRPA1 agonists, and it is known that such TRPA1 agonists exhibit an effect of increasing food intake to ameliorate decreased appetite (Patent Literature 10 and Non Patent Literature 1).

Citation List

Patent Literatures

Patent Literature 1: Japanese Unexamined Patent Application Publication No. 2018-177739

Patent Literature 2: International Application Japanese-Phase Publication No. 2017-518963

Patent Literature 3: Japanese Unexamined Patent Application Publication No. 2017-096785

Patent Literature 4: Japanese Unexamined Patent Application Publication No. 2014-210725

Patent Literature 5: Japanese Unexamined Patent Application Publication No. 2014-169240

Patent Literature 6: Japanese Unexamined Patent Application Publication No. 2014-076979

Patent Literature 7: Japanese Unexamined Patent Application Publication No. 2014-024810

Patent Literature 8: International Application Japanese-Phase Publication No. 2011-506289

Patent Literature 9: International Publication No. WO 2009/123080

Patent Literature 10: Japanese Unexamined Patent Application Publication No. 2020-109065

Non Patent Literatures

Non Patent Literature 1: Research Report of the Mishima Kaiun Memorial Foundation

Summary of Invention

Problems to be solved by the invention
The present invention aims to provide a novel agonist of TRPA1.
Means for solution of the problems
The present inventors carried out intensive studies to achieve the above object and found that a mushroom extract exhibits a TRPA1-activating effect (agonist action); as a result, the present invention was completed. Specifically, the present invention provides a composition for activating TRPA1, as described below, and also provides a composition for increasing food intake and/or ameliorating decreased appetite.

[1] A composition for activating transient receptor potential ankyrin 1 (TRPA1), comprising a mushroom extract.
[2] The composition according to [1], wherein the mushroom belongs to Basidiomycota or Ascomycota.
[3] The composition according to [1], wherein the extract contains lenthionine.
[4] The composition according to any one of [1]-[3], which is a food composition, a pharmaceutical composition, or an agrochemical composition.
[5] A composition for increasing food intake and/or ameliorating decreased appetite, comprising a mushroom extract.
[6] The composition according to [5], wherein the mushroom belongs to Basidiomycota or Ascomycota.
[7] The composition according to [5], wherein the extract contains lenthionine.
[8] The composition according to any one of [5]-[7], which is a food composition or a pharmaceutical composition.
[9] A composition for activating TRPA1, comprising lenthionine.
[10] A composition for increasing food intake and/or ameliorating decreased appetite, comprising lenthionine.

Advantageous Effects of Invention

According to the present invention, TRPA1 can be activated by a mushroom extract or by lenthionine; by means of this activating effect, appetite can be increased to boost food intake, and decreased appetite can be ameliorated.

Brief Description of Drawings

Fig. 1 shows changes in mean ± standard deviation from baseline responses to the question, "How hungry do you feel?" (* $p<0.05$ by t-test, indicating a significant difference relative to the group with control test food intake at the same time point).

Description of Embodiments

The invention is described in further detail below.

The composition of the present invention is used for activating TRPA1 and contains, as its active ingredient, a mushroom extract. Among compounds contained in the mushroom and/or among compounds produced from the mushroom during the process of preparing the extract, there are compounds having a TRPA1-activating effect. As confirmed specifically in the Examples described later, this TRPA1-activating effect has been detected in a variety of mushroom extracts regardless of the type of mushroom. Hence, it is not necessary to limit the type of mushroom, which may be either a Basidiomycete or an Ascomycete, or one or more mushrooms selected from among these groups, particularly edible mushrooms.

For example, the mushroom may be one belonging to the class Agaricomycetes such as the orders Agaricales, Auriculariales, or Polyporales, or one belonging to the class Pezizomycetes such as the Pezizales. Specific examples of Agaricales include mushrooms of the family Physalacriaceae such as enokitake, mushrooms of the family Pleurotaceae such as eringi and hiratake, mushrooms of the family Omphalotaceae such as shiitake, mushrooms of the family Lyophyllaceae such as bunashimeji, mushrooms of the family Strophariaceae such as nameko, and mushrooms of the family Agaricaceae such as the common mushroom (Agaricus bisporus). Specific examples of the order Auriculariales include mushrooms of the family Auriculariaceae such as kikurage. Specific examples of the order Polyporales include mushrooms of the family Sparassidaceae such as hanabiratake, and mushrooms of the family Meripilaceae such as maitake. Specific examples of the order Pezizales include mushrooms of the family Tuberaceae such as black truffles.

The amount of mushroom extract to be blended is not particularly limited as long as the TRPA1-activating effect is achieved; for example, it may be about 100 μg/mL to about 1000 mg/mL, in terms of the dry weight of the raw material relative to the total mass of the composition, or it may be about 10 mg/mL to about 100 mg/mL.

The mushroom extract can be prepared as appropriate by an extraction method commonly used in this technical field; for example, the mushroom extract may be a steam-distilled extract, a hot-water extract, an organic solvent extract, or a supercritical extract. As for the organic solvent used in preparing the organic solvent extract, it is possible to employ, without particular limitation, those commonly used in this technical field; examples include alcohols such as ethanol and propylene glycol, aldehydes, organic acids, esters, and ethers. The form of the mushroom extract is also not particularly limited; for example, it may be in liquid form (as an extract solution) or in powder/granular form.

**In** one embodiment, the extract contains an organosulfur compound having two or more, or three or more sulfur atoms, for instance lenthionine. Lenthionine, the compound also called 1,2,3,5,6-pentathiepane, has the following structure:

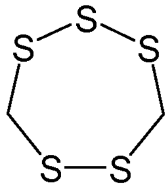.

Lenthionine is a compound known as a flavor component of shiitake mushrooms; it is hardly detected in intact shiitake mushrooms but can be generated during processing (that is, during the process of preparing the extract) if the shiitake tissue is damaged; for example, the lentinic acid in the shiitake may be acted upon by glutamyl transpeptidase and cysteine sulfoxide lyase.

In one embodiment, the composition for activating TRPA1 of the present invention is a food composition, a pharmaceutical composition, or an agrochemical composition. By means of its TRPA1-activating effect, the composition can be used to, for example, impart pungency to the target food, to increase food intake in a subject, to enhance the subject's appetite, to ameliorate the subject's decreased appetite, respiratory failure, ileus, or constipation, to boost the subject's energy metabolism to thereby prevent or improve obesity, or to repel pests.

The composition for activating TRPA1 of the present invention may further contain any additional inactive components commonly used in this technical field, such as pharmaceutically or nutritionally acceptable solvents, buffering agents, sweeteners, acidulants, flavorings, antifoaming agents, and antioxidants, as long as it does not impair the object of the present invention. The composition may also contain any additional active ingredients commonly used in this technical field, so long as this does not impair the object of the present invention; such an additional active ingredient may be one having a TRPA1-activating effect, or it may be an ingredient having another effect.

In another embodiment, the present invention relates to a composition for increasing food intake and/or ameliorating decreased appetite, which contains a mushroom extract as its active ingredient. The mushroom and its extract are the same as those described above for the composition for activating TRPA1 in the present invention.

Without wishing to be bound by any particular theory, it is known that TRPA1 agonists transmit peripheral information to the brain via sensory nerves, including afferent vagal nerves, thereby increasing food intake in the subject administered (see Non Patent Literature 1, and Patent Literature 10 if necessary); it is thus considered that the mushroom extract provided in the present invention, by activating TRPA1 through a similar mechanism, increases food intake and ameliorates decreased appetite.

In one embodiment, the composition for increasing food intake and/or ameliorating decreased appetite of the present invention is a food composition or a pharmaceutical composition. The composition can be used to increase the subject's food intake, to boost the subject's appetite, or to ameliorate the subject's decreased appetite. For this reason, the composition may be labeled, for example, as follows:

- For those who have no appetite
- For those who want to eat to their heart's content
- For those who want to build a bigger body
- For those who wish to maintain adequate meal portions, which might otherwise become insufficient
- For those who want to maintain motivation to eat.

The subject to which the composition for increasing food intake and/or ameliorating decreased appetite of the present invention is applied is not particularly limited; for example, it may be a human (specifically, a person in convalescence after an illness, an elderly person exhibiting age-related decrease in appetite, a child with poor growth, an athlete, or an individual with low stress tolerance) or a different species of mammal (a pet such as a dog or cat, or livestock such as a pig or cow).

The composition for increasing food intake and/or ameliorating decreased appetite of the present invention may further contain any additional inactive components commonly used in this technical field, such as pharmaceutically or nutritionally acceptable solvents, buffering agents, sweeteners, acidulants, flavorings, antifoaming agents, and antioxidants, as long as it does not impair the object of the invention. In addition, the composition may further contain any additional active ingredients commonly used in this technical field, as long as this does not impair the object of the invention; such an additional active ingredient may be one having an effect of increasing food intake or ameliorating decreased appetite, or it may be an ingredient having another effect.

In another embodiment, the present invention relates to a composition containing lenthionine; in particular, it concerns a composition for activating TRPA1, or a composition for increasing food intake and/or ameliorating decreased appetite. Lenthionine may be included as an active ingredient in the composition in its isolated compound form or in the form of an extract or a mixture. In one embodiment, lenthionine is included in the composition as a mushroom extract.

The concentration of lenthionine is not particularly limited as long as it exerts a TRPA1-activating effect, a food

intake-increasing effect, or a decreased-appetite-ameliorating effect; for example, it may be about 0.3 μM to about 3 mM relative to the total mass of the composition, and preferably about 30 μM to about 300 μM.

Particular embodiments of the composition containing lenthionine according to the present invention are the same as those described above for the composition for activating TRPA1 of the present invention and for the composition for increasing food intake and/or ameliorating decreased appetite of the present invention.

In another embodiment, the present invention also relates to a method of activating TRPA1, a method of increasing food intake, and/or a method of ameliorating decreased appetite, comprising administering to a subject in need thereof a mushroom extract, lenthionine, or a composition containing a mushroom extract and/or lenthionine. Particular embodiments of the mushroom extract, lenthionine, or the composition containing a mushroom extract and/or lenthionine used in the method of the present invention are as described above for the composition for activating TRPA1 of the present invention and for the composition for increasing food intake and/or ameliorating decreased appetite of the present invention.

In another embodiment, the present invention also relates to the use of a mushroom extract and/or lenthionine for manufacturing a composition for activating TRPA1, for increasing food intake, and/or for ameliorating decreased appetite. Particular embodiments of the various compositions manufactured by the use of the present invention are as described above for the composition for activating TRPA1 of the present invention and for the composition for increasing food intake and/or ameliorating decreased appetite of the present invention.

In another embodiment, the present invention also relates to a TRPA1 activator, a food intake-increasing agent, and/or a decreased-appetite-ameliorating agent, which contain a mushroom extract and/or lenthionine. Particular embodiments of the TRPA1 activator, the food intake-increasing agent, and the decreased-appetite-ameliorating agent of the present invention are as described above for the composition for activating TRPA1 of the present invention and for the composition for increasing food intake and/or ameliorating decreased appetite of the present invention.

In another embodiment, the present invention also relates to the non-therapeutic use of a mushroom extract and/or lenthionine as a TRPA1 activator, as a food intake-increasing agent, and/or as a decreased-appetite-ameliorating agent. Particular embodiments of the TRPA1 activator, the food intake-increasing agent, and the decreased-appetite-ameliorating agent used non-therapeutically are as described above for the composition for activating TRPA1 of the present invention and for the composition for increasing food intake and/or ameliorating decreased appetite of the present invention.

Hereinafter, the present invention is explained in detail with reference to the following Examples; however, the scope of the present invention is not limited to these Examples.

[Examples]

1. Preparation Examples

(1) Preparation of Mushroom Extract Solutions

Five grams of each dried mushroom (※) were ground into powder using a grinder, then 95 g of water was added and stirred with a stirrer for 10 minutes at room temperature. Next, 100 mL of dichloromethane was added and the mixture was stirred for an additional 30 minutes. After stirring, 100 mL of Celite was added to the solution and stirred with a glass rod until it formed a slurry. This dispersion was filtered through a suction funnel (Celite filtration). The Celite and residue remaining on the funnel were scraped off with a spatula, 100 mL of dichloromethane was added again, and the mixture was filtered once more.

(※) Mushrooms used: enoki, eringi, kikurage, shiitake, shimeji, black truffle, nameko, hanabiratake, hiratake, maitake, black mushroom, and white mushroom.

The collected filtrate was transferred to a separatory funnel; 100 mL of pure water was added and stirred, then the water layer was discarded to wash the sample by liquid-liquid partition. This washing step was repeated twice more. The washed dichloromethane layer was collected into an Erlenmeyer flask, and anhydrous sodium sulfate was added until it no longer aggregated, in order to remove water.

The dehydrated sample solution was transferred to a round-bottom flask, and the solvent was removed with a rotary evaporator until the volume was reduced to less than 5 mL. The concentrated sample was collected and brought up to 5 mL with dichloromethane, thereby obtaining each mushroom extract solution (1 g/mL in terms of dry weight of the raw material). This extract solution was dispensed in 1 mL portions into vials and stored at -80°C until use.

(2) Detection of Lenthionine

The dichloromethane extract of shiitake prepared in item 1 above was analyzed by GC-MS using a conventional

method; multiple peaks were detected that included MS fragments characteristic of sulfur-containing compounds. From the fragment patterns of each peak, lenthionine was detected.

2. Examples of TRPA1 Activation Tests

(1) Preparation of TRPA1 Expression Vector and Its Stable Expression Cell Line

A DNA coding for human TRPA1 (hTRPA1) (GenBank accession number: NM_007332.3) was inserted into the pcDNA5/TO vector (Invitrogen) to create an expression vector. This expression vector was transfected into T-REx™-293 cells (Invitrogen) by a conventional method using Lipofectamine LTX (Invitrogen). Using 10% fetal bovine serum (FBS, Cytiva), 400 μ/mL hygromycin B, and 5 μg/mL blasticidin S, which were added to Dulbecco's Modified Eagle Medium (DMEM, Nacalai Tesque) as drug selection medium, the transfectants were cultured by a conventional method and cloned to obtain an hTRPA1 stable expression cell line.

(2) Intracellular $Ca^{2+}$ Imaging Assay 1

Two hundred microliters of each mushroom extract solution prepared in item 1 (1) was dispensed and the solvent was removed under vacuum using a desiccator. The dried residue left in the container was dissolved in 1 mL of assay buffer (Hank's Balanced Salt Solution without calcium and magnesium ions, containing 1 mM CaCl2 dihydrate, 20 mM HEPES, and 0.1% BSA). This was centrifuged (200 rpm, 1 minute) to remove insoluble material, thereby preparing a test mushroom extract solution at 0.2 g/mL in terms of the dry weight of the raw material.

Next, an hTRPA1 stable expression cell line whose hTRPA1 expression had been induced in advance by a conventional method was prepared in a 96-well clear-bottom plate; after washing each well with PBS, 50 μL of assay buffer (Hank's Balanced Salt Solution without calcium and magnesium ions, containing 1 mM CaCl2 dihydrate, 20 mM HEPES, 0.1% BSA, and 4 μM Calbryte™ 520 AM (manufactured by AAT BioQuest)) was added and incubated for 45 minutes at 33°C. After washing with assay buffer, 100 μL of assay buffer was added to each well.

This plate was set in FlexStation3 (Molecular Devices) and the fluorescence intensity (excitation at 490 nm, emission at 525 nm) was measured every 2 seconds at 31°C; 30 seconds after the start of measurement, 100 μL of assay buffer containing the negative control DMSO (final concentration: 20 μL/mL), the positive control allyl isothiocyanate (AITC) (AITC was dissolved in DMSO; final concentration: 30 μM), each mushroom extract solution described above, or lenthionine (manufactured by Ambeed, Inc.) (lenthionine was dissolved in DMSO; final concentration: 50 μM) was added. In addition, to determine the reference for fluorescence intensity, wells to which assay buffer containing ionomycin (final concentration: 10 μM after addition) was added were prepared instead of the test samples.

Fluorescence intensity measurement was continued until 120 seconds from the start; the TRPA1-activating ability of the test samples and so forth was calculated using the formula below. The results are shown in Table 1.

$$\text{Activation rate (\%)} = (F_{max[s]} - F_0)/(F_{max[I]} - F_0) \times 100$$

$F_{max[s]}$: maximum fluorescence intensity after adding the test sample or positive control
$F_0$: mean fluorescence intensity for 20 seconds from the start of measurement (baseline)
$F_{max[I]}$: maximum fluorescence intensity after adding ionomycin

Table 1: TRPA1 Activation Rate (Mean±Standard Error)

| Test Sample | Activation Rate (%) |
|---|---|
| AITC 30 μM (n=6) | 106±1.89 |
| Mushroom Extracts | |
| Enoki (n=5) | 90.5±1.00 |
| Eringi (n=6) | 124±3.49 |
| Kikurage (n=6) | 57.6±2.31 |
| Shiitake (n=6) | 123±3.14 |
| Shimeji (n=6) | 135±4.22 |
| Black truffle (n=6) | 122±1.76 |
| Nameko (n=6) | 119±1.07 |
| Hanabiratake (n=6) | 126±2.58 |

(continued)

| Test Sample | Activation Rate (%) |
|---|---|
| Hiratake (n=6) | 147±2.26 |
| Maitake (n=6) | 120±2.18 |
| Black mushroom (n=6) | 104±2.86 |
| White mushroom (n=6) | 91.3±3.98 |
| Lenthionine 50 μM (n=6) | 93.0±3.70 |
| DMSO (n=6) | 1.01±0.0549 |

All mushroom extracts activated TRPA1. Consequently, it was shown that a mushroom extract has a TRPA1-activating effect. In addition, lenthionine alone, contained in the shiitake extract, also activated TRPA1. Thus, it was indicated that lenthionine is among the active ingredients responsible for TRPA1 activation in the shiitake extract.

(3) Intracellular $Ca^{2+}$ Imaging Assay 2

Aside from using samples in which the concentrations of AITC or lenthionine were set stepwise in the range of 0.01 μM-300 μM, the procedure was the same as in (2) above; the TRPA1-activating abilities of each sample were measured. The results are shown in Table 2.

Table 2: TRPA1 Activation Rate (Mean±Standard Error, n=6)

| | Activation Rate (%) | |
|---|---|---|
| Concentration | AITC | Lenthionine |
| 300 μM | 119±2.74 | 118±2.65 |
| 100 μM | 117±2.57 | 104±1.94 |
| 50 μM | 105±1.86 | 92.6±3.83 |
| 10 μM | 85.2±3.76 | 72.5±2.88 |
| 5 μM | 64.0±4.13 | 53.8±2.29 |
| 1 μM | 13.0±1.79 | 22.3±1.04 |
| 0.5 μM | 6.19±0.32 | 10.2±0.82 |
| 0.1 μM | 5.20±0.19 | 2.42±0.14 |
| 0.01 μM | 4.02±0.50 | 2.64±0.19 |

Lenthionine activated TRPA1 in a concentration-dependent manner, at similar intensity to AITC. Hence, it was confirmed that lenthionine has a TRPA1-activating effect.

(4) Intracellular $Ca^{2+}$ Imaging Assay 3

Samples of 30 μM AITC, 100 μM or 5 μM lenthionine, or DMSO were used; the test was performed either in the presence of the TRPA1-specific antagonist A-967079 (1 μM) or in the absence thereof (DMSO: 20 μL/mL); apart from this, the procedure was the same as in (3) above, and the TRPA1-activating abilities of each sample were measured. The results are shown in Table 3.

Table 3: TRPA1 Activation Rate (Mean±Standard Error, n=6)

| Test Sample | Activation Rate (%) | |
|---|---|---|
| | No Antagonist | With Antagonist |
| AITC 30 μM | 156±1.38 | 1.22±0.06* |
| Lenthionine | | |
| 100 μM | 133±1.36 | 1.61±0.37* |
| 5 μM | 64.6±1.38 | 1.46±0.14* |

(continued)

| Test Sample | Activation Rate (%) | |
|---|---|---|
| | No Antagonist | With Antagonist |
| DMSO | 3.35±0.0748 | 1.40±0.0786 |
| *p<0.05 (t-test, significant difference compared to the test group without antagonist) | | |

The TRPA1-activating effect of lenthionine was inhibited by the TRPA1-specific antagonist; thus, the TRPA1-activating effect of lenthionine was confirmed once again.

3. Test 1 for Ameliorating Decreased Appetite

Male ICR mice were kept in individual cages with ALPHA-dri (Shepherd Specialty Papers) as bedding, and they were fed a powdered diet (CE-2, CLEA Japan) using a powder feeder for mice (SN-950, Shinano Seisakusho Co., Ltd.), with ad libitum feeding and free access to water, for at least one week for acclimatization. During the few days prior to the experiment, daily handling and gavage training using a feeding needle (FG4202, Fuchigami Kikai) were performed.

As test samples, the following were prepared for each of carrier solution (containing, by volume, 5% dimethyl sulfoxide, 1% Tween80, and 94% corn oil) and lenthionine solution (carrier solution containing 0.94 mg lenthionine per 1 mL): one containing the TRPA1-specific antagonist A-967079 (1.0 μmol of A-967079 per 1 mL of the carrier solution) and one not containing it; one of these was administered to 11- to 12-week-old mice under ad libitum feeding conditions by a single gastric gavage at 9:20 a.m. at 10 mL/kg body weight. The dose of lenthionine in molar terms was 50 μmol/kg body weight. Immediately after the single gavage, at 9:30 a.m., the powdered feeder inside the cage was replaced with a new feeder loaded with powdered diet whose mass had been measured beforehand; this time point was set as the start of the feeding experiment. Thirty minutes after the start of the feeding experiment, the mass of the feeder and any spilled feed in the cage was measured, and the food intake (g) was calculated (n=10). Table 4 shows each group's food intake and the results of multiple comparisons by Dunn-Bonferroni test.

Table 4: Comparison of Food Intake in Mice (Mean±Standard Error, n=10)

| Test Sample | Food Intake (g) | |
|---|---|---|
| | No Antagonist | With Antagonist |
| Carrier Solution | 0.024±0.013 | 0.016±0.012 |
| Lenthionine | 0.117±0.035 | 0.025±0.023 |
| * p<0.05 (multiple comparisons, significant difference compared to the corresponding carrier solution group) | | |

The mice's food intake increased when the lenthionine solution was administered (see the "No Antagonist" column in Table 4); however, when the TRPA1-specific antagonist A-967079 was administered, no increase in food intake was observed (see the "With Antagonist" column in Table 4). Thus, it was demonstrated that lenthionine causes an increase in appetite and that this increase in appetite is mediated by TRPA1 activation. Therefore, it is considered that lenthionine or a mushroom extract having a TRPA1-activating effect can increase appetite to boost food intake and ameliorate decreased appetite by activating TRPA1.

4. Test 2 for Ameliorating Decreased Appetite

(1) Preparation of Test Food

Ten mL of citrate-phosphate buffer was added to 1 g of dried shiitake mushroom powder, and the mixture was stirred for 10 minutes. Then, 10 mL of salad oil was added and stirred overnight. This was centrifuged, and only the salad oil layer was collected to prepare shiitake oil. Twenty-five mL of the shiitake oil was dissolved in 25 mL of hot water at about 60°C to prepare a shiitake oil test food. As a control test food, salad oil alone dissolved in hot water was used in place of shiitake oil. When the shiitake oil was analyzed by gas chromatography-mass spectrometry (GC/MS), a peak corresponding to lenthionine was detected, confirming that the extracted oil contained lenthionine.

(2) Method of Evaluating Appetite

Appetite-related indicators were evaluated using a Japanese version of the Appetite Questionnaire (Nagai et al., Journal of Japan Society for the Study of Obesity, Vol. 18, No. 1, pp. 39-51, 2012), which employs a visual analog scale (VAS). Simply put, for the question "How hungry do you feel?," the subject marks a point on a 100-mm horizontal line (VAS) labeled with the minimum at the left end and the maximum at the right end, according to their current feeling; the examiner measures the distance from the left end to the mark; the value measured immediately after ingestion of the test food was set as the baseline (zero), and the change from baseline (difference) was recorded.

(3) Test Procedure

On the test day, subjects woke up by 6:00 a.m. and did not eat or drink except for water intake until 9:00 a.m. At 9:00 a.m., they evaluated their appetite using the VAS. They then consumed the designated food (Calorie Mate Block (maple flavor), manufactured by Otsuka Pharmaceutical Co., Ltd.), 4 bars, and evaluated their appetite again immediately afterward. Starting from 9:00 a.m., appetite was evaluated every 30 minutes; after the evaluation at 11:00 a.m., the subjects consumed the test food (either control or shiitake oil), then evaluated appetite again immediately afterward. They wore a nose clip for 30 minutes from immediately after consuming the test food until the 11:30 a.m. appetite evaluation. At 12:30 p.m., they evaluated appetite again, consumed the designated food, and performed another immediate evaluation of appetite. Finally, they evaluated appetite at 1:00 p.m. and concluded the test.

Seven healthy Japanese men and women aged 20-69, who go about their daily lives without difficulty, participated in the test. Two test sessions in total were conducted, and each participant acted as a subject for both sessions. The subjects were not informed which test food they would eat, but in one session they consumed the shiitake oil test food, and in the other session they consumed the control test food. The second test session was conducted at least 3 days after the first session. Mean $\pm$ standard deviation of the responses to each question (the VAS difference) was calculated and graphed to observe the changes in the values.

(4) Test Results

As shown in Fig. 1, in the group that consumed the shiitake oil test food, the sensation of hunger was stronger compared to the group that consumed the control test food. In particular, at 30 minutes after consuming the test food, a significant difference was observed by t-test at a significance level of 0.05; hence, it was demonstrated that shiitake oil increases hunger and boosts appetite. Therefore, it is considered that a mushroom extract having a TRPA1-activating effect or the lenthionine contained therein actually exerts a food intake-increasing effect and a decreased-appetite-ameliorating effect.

From the above, it was found that TRPA1 can be activated by a mushroom extract or lenthionine, and that appetite can be increased to boost food intake and ameliorate decreased appetite by means of this activating effect.

## Claims

1. A composition for activating transient receptor potential ankyrin 1 (TRPA1), comprising a mushroom extract.

2. The composition according to claim 1, wherein the mushroom belongs to Basidiomycota or Ascomycota.

3. The composition according to claim 1, wherein the extract contains lenthionine.

4. The composition according to any one of claims 1-3, which is a food composition, a pharmaceutical composition, or an agrochemical composition.

5. A composition for increasing food intake and/or ameliorating decreased appetite, comprising a mushroom extract.

6. The composition according to claim 5, wherein the mushroom belongs to Basidiomycota or Ascomycota.

7. The composition according to claim 5, wherein the extract contains lenthionine.

8. The composition according to any one of claims 5-7, which is a food composition or a pharmaceutical composition.

9. A composition for activating TRPA1, comprising lenthionine.

**10.** A composition for increasing food intake and/or ameliorating decreased appetite, comprising lenthionine.

FIG.1

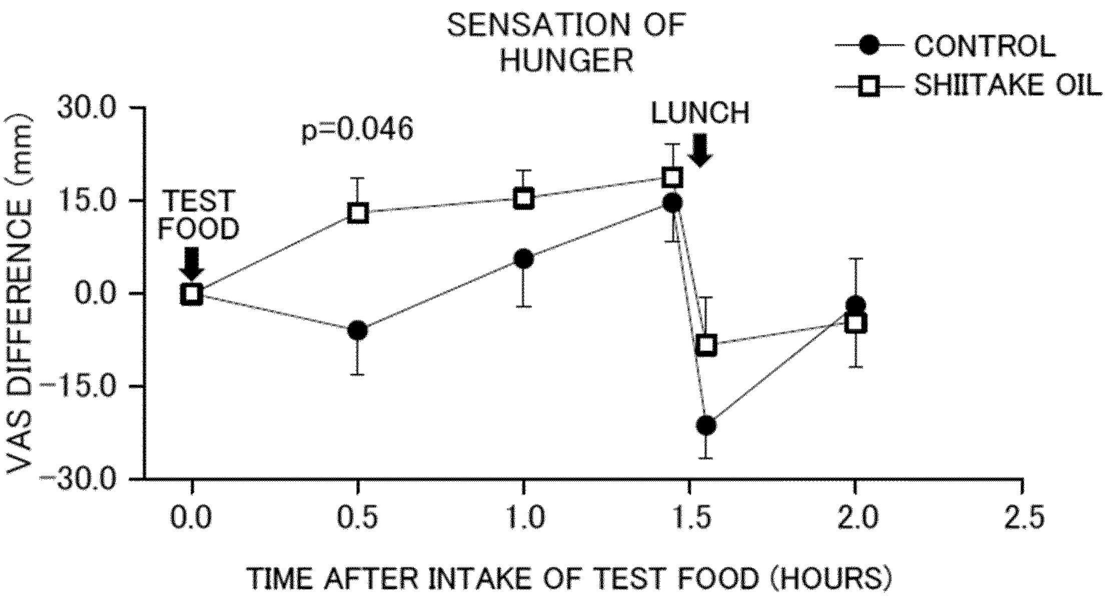
SENSATION OF
HUNGER
CONTROL
SHIITAKE OIL
30.0
15.0
0.0
−15.0
−30.0
VAS DIFFERENCE (mm)
p=0.046
TEST
FOOD
LUNCH
0.0
0.5
1.0
1.5
2.0
2.5
TIME AFTER INTAKE OF TEST FOOD (HOURS)

## INTERNATIONAL SEARCH REPORT

International application No.
**PCT/JP2023/039296**

**A. CLASSIFICATION OF SUBJECT MATTER**

***A23L 33/10***(2016.01)i; ***A61K 36/062***(2006.01)i; ***A61K 36/07***(2006.01)i; ***A61P 1/14***(2006.01)i; ***A61P 43/00***(2006.01)i
FI: A23L33/10; A61K36/07; A61K36/062; A61P1/14; A61P43/00 111

According to International Patent Classification (IPC) or to both national classification and IPC

**B. FIELDS SEARCHED**

Minimum documentation searched (classification system followed by classification symbols)

A23L33/10; A61K36/062; A61K36/07; A61P1/14; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS/FSTA/AGRICOLA (STN)

**C. DOCUMENTS CONSIDERED TO BE RELEVANT**

| Category* | Citation of document, with indication, where appropriate, of the relevant passages | Relevant to claim No. |
|---|---|---|
| X | KAIMOTO, T. et al. Involvement of transient receptor potential A1 channel in algesic and analgesic actions of the organic compound limonene. European Journal of Pain. 2016, vol. 20, no. 7, pp. 1155-1165<br>abstract | 1-2, 4 |
| Y | | 5-6, 8 |
| A | | 3, 9 |
| Y | JP 2020-109065 A (UNIV JICHI MEDICAL) 16 July 2020 (2020-07-16)<br>claims, examples | 5-6, 8 |
| A | | 3, 9 |
| X | JP 2014-169240 A (OGAWA & CO LTD) 18 September 2014 (2014-09-18)<br>claims, paragraphs [0030], [0038]-[0039] | 1-2, 4 |
| Y | | 5-6, 8 |
| A | | 3, 9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 December 2023** | **23 January 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/039296**

**C. DOCUMENTS CONSIDERED TO BE RELEVANT**

| Category* | Citation of document, with indication, where appropriate, of the relevant passages | Relevant to claim No. |
|---|---|---|
| X | JP 2014-210725 A (OGAWA & CO LTD) 13 November 2014 (2014-11-13) claims, paragraphs [0030], [0038]-[0039] | 1-2, 4 |
| Y | | 5-6, 8 |
| A | | 3, 9 |
| X | JP 2004-135556 A (MORIYAMA, Yuji) 13 May 2004 (2004-05-13) paragraphs [0002], [0037] | 5-8, 10 |
| A | | 3, 9 |
| X | KR 10-2008-0090630 A (JANG, Chan-Hong) 09 October 2008 (2008-10-09) paragraph [0018] | 5-8, 10 |
| A | | 3, 9 |

Form PCT/ISA/210 (second sheet) (January 2015)

**INTERNATIONAL SEARCH REPORT**
**Information on patent family members**

International application No.
**PCT/JP2023/039296**

| Patent document cited in search report | Publication date (day/month/year) | Patent family member(s) | Publication date (day/month/year) |
|---|---|---|---|
| JP 2020-109065 A | 16 July 2020 | (Family: none) | |
| JP 2014-169240 A | 18 September 2014 | (Family: none) | |
| JP 2014-210725 A | 13 November 2014 | (Family: none) | |
| JP 2004-135556 A | 13 May 2004 | (Family: none) | |
| KR 10-2008-0090630 A | 09 October 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018177739 A
- JP 2017518963 A
- JP 2017096785 A
- JP 2014210725 A
- JP 2014169240 A
- JP 2014076979 A
- JP 2014024810 A
- JP 2011506289 A
- WO 2009123080 A
- JP 2020109065 A


### Non-patent literature cited in the description


- **NAGAI et al.** *Journal of Japan Society for the Study of Obesity*, 2012, vol. 18 (1), 39-51